# FASCICULE DE BREVET EUROPEEN

(11) **EP 1 824 558 B1**
(45) Date de publication et mention de la délivrance du brevet: **06.11.2013**
(21) Numéro de dépôt: 05785600.7
(22) Date de dépôt: 07.09.2005
(51) Int. Cl.: A61N 1/04, A61N 1/32

(54) **APPAREIL POUR L'ELECTRO-INHIBITION DES MUSCLES DE LA FACE**
VORRICHTUNG FÜR DIE ELEKTRO-INHIBITION DER GESICHTSMUSKELN
APPARATUS FOR THE ELECTRO-INHIBITION OF FACIAL MUSCLES

(30) Priorité: 14.12.2004 EP 04447276
(43) Date de publication de la demande: 29.08.2007
(73) Titulaire: CEFALY Technology SPRL, 4040 Herstal (BE)
(72) Inventeur: RIGAUX, Pierre, B-4020 LIEGE (BE)
(74) Mandataire: Lerho, Marc J. A.
(86) Numéro de dépôt international: PCT/BE2005/000135
(87) Numéro de publication internationale: WO 2006/063417

(56) Documents cités:
- WO-A-00/71075
- WO-A-01/03638
- US-A- 3 709 228
- US-A- 5 496 363
- US-A- 5 527 357
- US-A- 5 913 836
- US-B1- 6 198 955

## Description

### Objet de l'invention

La présente invention se rapporte à un appareil d'inhibition électrique ou "électro-inhibition" permettant d'obtenir le relâchement de certains muscles de la partie supérieure du visage.

### Arrière-plan technologique et état de la technique

Les rides peuvent être dues à une multitude de facteurs comme l'âge, les dommages causés par le soleil, etc., mais elles sont surtout dues aux mimiques, c'est-à-dire aux contractions musculaires répétées (expression de joie, de colère, de tristesse, de peur, d'étonnement, etc.). On parle donc de "rides d'expression". Ces rides qui apparaissent principalement dans la moitié supérieure du visage sont dues aux interactions entre la peau et les muscles sous-jacents. On connaît notamment les rides verticales du front, les rides du coin des yeux appelées "pattes d'oie", les rides horizontales du front, etc.

Ainsi, les rides du front, de même que la plupart des céphalées chroniques, communément appelés "maux de tête" ou "migraines", ont pour cause une tension prolongée induisant une contracture chronique soit de l'un des trois muscles de la partie supérieure de la face qui sont le muscle frontal *(frontalis),* le double muscle sourcilier *(corrugator)* et le muscle pyramidal du nez *(procerus),* soit encore de plusieurs d'entre eux en même temps.

Le muscle pyramidal est un petit faisceau musculaire charnu situé sur la partie supérieure du dos du nez et entre les yeux. Il s'étend entre le fascia recouvrant la partie inférieure de l'os propre du nez et la face profonde de la peau de la région inter-sourcilière. Il provoque une attraction des sourcils vers le bas et la ligne médiane, en créant des replis sur la racine du nez. Il intervient dans le froncement des sourcils, l'expression de la concentration ou tout simplement pour réduire l'éblouissement en cas de lumière vive.

Le muscle frontal est un muscle plat situé sous la peau du front. Il naît du bord antérieur de l'aponévrose épicrânienne, ses fibres charnues descendent et s'attachent à la face profonde de la peau des régions sourcilière et inter-sourcilière. Il élève la peau des sourcils et produit les rides et replis horizontaux du front.

Les deux muscles sourciliers sont étendus de part et d'autre de la glabelle le long de la partie interne de l'arcade sourcilière. Ils naissent de l'extrémité interne de l'arcade sourcilière et se dirigent en dehors le long de l'arcade pour se terminer à la face profonde de la peau des sourcils. Ils attirent les sourcils vers la ligne médiane et sont responsable des rides et replis cutanés verticaux de la glabelle.

L'obtention d'un relâchement de ces muscles permettrait donc de lutter contre les céphalées et les rides de tension. De plus, un relâchement de ces trois muscles de la partie supérieure du visage est susceptible de permettre à l'activité des muscles de la zone occipitale de prédominer. Ces derniers sont le muscle occipital *(occipitalis)* et les deux muscles auriculaires postérieurs *(auricularis posterior).*

Ce changement d'équilibre entre muscles de la face et muscles de l'occiput à l'avantage de ces derniers est susceptible de produire un étirement de la peau de la face, avec effet de lifting physiologique, qui améliore l'apparence du visage.

On connaît actuellement différents traitements de correction des rides disgracieuses telles que rides du front, de la glabelle ou pattes d'oie, apparaissant sur les peaux vieillissantes.

Certains fabricants de crèmes de soins préconisent une gestuelle de massage appropriée pour décontracter les muscles faciaux. Cependant, on peut douter de l'efficacité d'un tel traitement, surtout lorsque les rides d'expression sont déjà bien marquées.

Il existe également des traitements d'injection de produits à action temporaire tel que l'acide hyaluronique ou la toxine botulinique de type A, dérivée de la bactérie *Clostridium botulinum* (connue sous le nom commercial Botox^{®}). Le Botox^{®} est injecté en doses faibles dans un muscle spécifique qui devient paralysé et ne peut plus dès lors se contracter. Ces produits ont une durée d'action de quelques mois.

On injecte également des implants permanents à base de microbilles en plastique ou des implants tubulaires en PTFE, ainsi que du collagène ou de la silicone. La longévité des implants est de plusieurs années. Il s'agit d'une chirurgie éventuellement réversible.

Ces techniques d'injection présentent toutefois un certain nombre d'effets secondaires tels que risque de développer une allergie, oedème après injection, risque d'infection, rougeurs, ecchymoses, sensations de gêne, etc.

De plus, les injections de Botox^{®} se retrouvent dans la circulation générale du patient. Un effet secondaire spécifique est notamment une observation de paralysie indésirable des muscles de la déglutition (dysphagie).

Par ailleurs, l'électro-stimulation musculaire est bien connue. Ainsi, par exemple, le brevet américain US-A-4,957,480 décrit une méthode de tonification des muscles et tissus du visage par stimulation des nerfs moteurs et contraction consécutive des muscles de la face, par application de courants galvaniques d'amplitude, de fréquence et de polarité prédéterminés au travers d'électrodes humidifiées au moyen d'une solution liquide de particules chargées positivement et négativement, en vue de son introduction dans les tissus pour nourrir les muscles et les tissus du visage environnants. On sait, à partir de ce document, qu'il est possible de cette manière, par un positionnement particulier des électrodes directement sur le muscle, d'obtenir une fatigue, et partant une relaxation, par exemple du muscle frontal. Les courants sont compris entre 300 et 640 µA avec une fréquence comprise entre 30 et 99 Hz, avec une polarisation alternée et une durée d'application comprise entre 1 et 4 secondes, voire même 10 secondes. Les paramètres électriques utilisés génèrent une fatigue de travail, semblable à la fatigue volontaire du muscle, qui ne produit qu'une relaxation peu marquée sinon nulle. Il s'agit du cadre classique de l'électro-stimulation avec des fréquences inférieures à 100 Hz pour produire des contractions courtes.

Le brevet américain US-A-3,709,228 décrit un appareil d'électro-stimulation des nerfs, et partant des muscles, de la face d'un utilisateur, comprenant un support reposant sur le nez et les oreilles et portant des bras déformables pouvant s'étendre dans des directions opposées et porteurs à leur extrémité d'électrodes de contact avec la peau. Une première électrode est positionnée en vue de stimuler la branche frontale du nerf facial. Une deuxième électrode est positionnée en vue de stimuler la branche maxillaire du nerf facial. Enfin, une troisième électrode est positionnée en vue de stimuler la branche mandibulaire du nerf facial. Ce document ne suggère pas un positionnement d'électrode au niveau de l'espace inter-sourcilier.

### Buts de l'invention

La présente invention vise à fournir un appareil permettant d'obtenir, par l'action d'un courant électrique, un relâchement d'un ou plusieurs muscles de la partie supérieure du visage, en particulier le muscle frontal (*frontalis*), les deux muscles sourciliers (*corrugator*) et le muscle pyramidal du nez (*procerus*).

### Principaux éléments caractéristiques de l'invention

L'objet de la présente invention, énoncé dans la revendication 1, concerne un dispositif d'électro-inhibition de muscles de la face comprenant :
- un support au niveau de la tête d'un utilisateur,
- deux électrodes de contact aptes à être disposées de chaque côté de la partie supérieure du nez, au niveau de la glabelle, lesdites électrodes appartenant à une structure unique d'électrode
- un circuit électronique pour la création d'impulsions électriques basse tension au niveau desdites électrodes et une alimentation électrique à courant continu,
   caractérisé en ce que:
- la structure unique d'électrode comprend une pièce de tissu servant de support auxdites électrodes, ces dernières étant couvertes sur leur face interne, c'est-à-dire la face devant être en contact avec la peau de l'utilisateur, par un gel conducteur autocollant et
- les deux électrodes se présentent sous la forme de deux zones argentées conductrices qui imprègnent ledit tissu, celui-ci étant conducteur aussi bien sur sa face interne que sur sa face externe, c'est-à-dire la face en contact avec des patins de l'appareil électrique.

Les revendications 2 à 12 précisent des modes d'exécution préférés du dispositif de la revendication 1.

### Brève description des figures

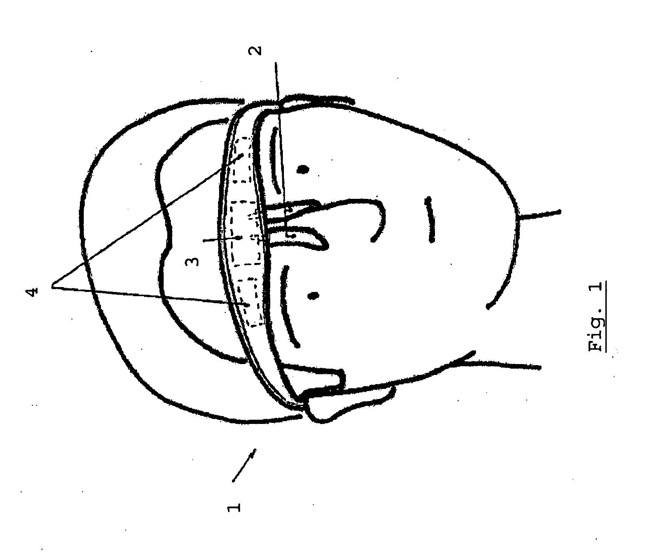

La figure 1 représente schématiquement une vue avant du dispositif d'électro-inhibition selon une première forme d'exécution préférée de la présente invention, placé sur la tête d'un utilisateur.

La figuré 2 représente une vue arrière du dispositif de la figure 1.

La figure 3 représente une vue du dessus pour le dispositif de la figure 1.

La figure 4 représente une vue en coupe et du dessus de la partie avant du dispositif de la figure 1.

La figure 5 représente schématiquement une vue avant (sans l'électrode) du dispositif d'électro-inhibition selon une deuxième forme d'exécution préférée de la présente invention, placé sur la tête d'un utilisateur.

La figure 6 représente une vue arrière du dipositif de la figure 5, ainsi qu'une vue de détail de l'électrode double placée sur son support.

La figure 7 représente différentes vues de l'électrode de la figure 6.

### Description de plusieurs formes d'exécution préférées de l'invention

### Méthode

Le principe innovant à la base de l'invention est de produire une inhibition des trois muscles précités dans la partie supérieure de la face au moyen de l'application d'un courant électrique sur un seul de ces muscles, à savoir le pyramidal. Ainsi le placement spécifique des électrodes permet, en inhibant le muscle pyramidal, d'obtenir des actions réflexes de relâchement sur les autres muscles de la partie supérieure du visage. Il s'agit donc d'un appareil visant l'effet inverse de celui recherché dans l'électro-stimulation, puisque le but recherché est le relâchement des muscles et non leur contraction.

### Description de la technique

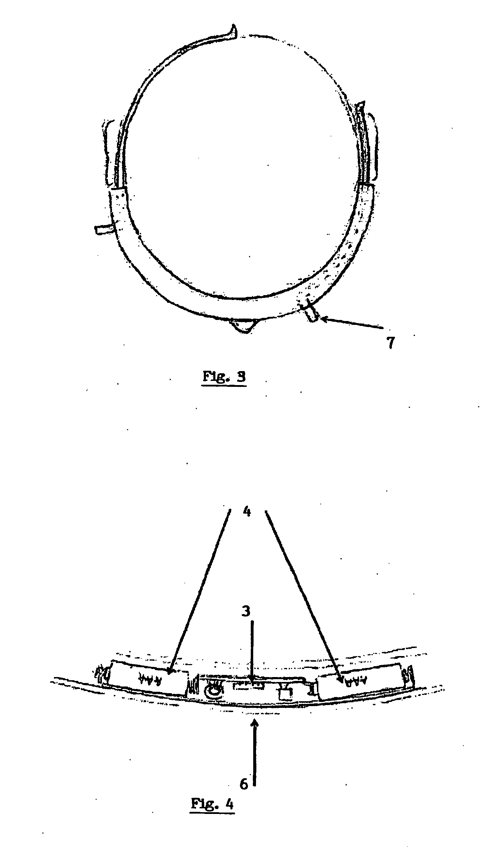

Selon l'invention représentée sur les figures 1 à 4, deux petites électrodes 2 sont positionnées parallèlement l'une à l'autre au niveau de l'espace inter-sourcilier appelé glabelle *(glabella),* de façon à faire passer le courant électrique d'inhibition au travers du muscle pyramidal du nez. Les électrodes 2 sont supportées par un serre-tête 1 .

Ces électrodes 2 ont essentiellement une forme rectangulaire dont la taille est plus ou moins de 25 mm sur 5 mm et sont seulement écartées l'une de l'autre par une distance de 3 à 15 mm.

L'avantage de ce placement particulier des électrodes est double :
- simplicité : avec seulement deux électrodes et un seul canal de stimulation on obtient l'effet de relaxation sur les trois muscles-cibles en même temps grâce aux mécanismes physiologiques décrits plus loin (fatigue électrique, réflexe d'inhibition et excitation des afférences tendineuses) ;
- confort : le très faible écartement entre les deux électrodes (3 à 15 mm) permet de limiter les sensations désagréables en réduisant le plus possible le volume des tissus soumis au courant électrique ^{(1,2)}. En effet, ce faible écartement des électrodes a pour double conséquence, d'une part, qu'une très petite surface de peau est soumise au courant électrique et, d'autre part, une très faible pénétration du courant en profondeur, celui-ci circulant donc préférentiellement dans les couches superficielles de l'épiderme sans exciter les terminaisons nerveuses du périoste, très sensibles à la douleur.

Le courant appliqué aux électrodes 2 est constitué d'impulsions électriques connues pour être capables de déclencher des potentiels d'action (PA) au niveau des nerfs moteurs. Il s'agit d'impulsions rectangulaires d'une largeur comprise entre 30 et 100 *µ*s et d'une intensité allant de 0 à 30 mA ⁽³⁾. L'utilisation de ce type d'impulsions conduit à une moindre excitation des fibres de la douleur que l'utilisation d'impulsions plus longues. Mais tout autre type d'impulsion suffisante pour déclencher un PA sur des motoneurones pourrait être employé.

Une caractéristique essentielle de l'invention est que le courant appliqué utilise avantageusement des impulsions présentant une fréquence supérieure à 100 Hz, ce qui génère une fatigue électrique très rapide du muscle pyramidal ^{(4, 5)}. Il se produit grâce à cette excitation continue des nerfs moteurs une extinction rapide de l'activité électrique des fibres musculaires innervées par les nerfs moteurs excités ⁽⁶⁾. Cette fréquence d'impulsion étant maintenue constante, en quelques dizaines de secondes, le muscle pyramidal devient inexcitable, perd son tonus et ainsi se relâche totalement ⁽⁷⁾.

L'excitation des motoneurones du muscle pyramidal produit également un relâchement de son muscle antagoniste -le muscle frontal- via le réflexe d'inhibition réciproque ⁽⁸⁾. En effet, les impulsions électriques au niveau d'un muscle excitent non seulement les motoneurones de ce muscle mais aussi les fibres afférentes proprioceptives de ce muscle. L'excitation de ces dernières inhibe les motoneurones du muscle antagoniste ^{(9,10)}.

De plus, les électrodes 2 étant situées au niveau des tendons internes des muscles sourciliers, le courant excite les afférences nerveuses de ces tendons, c'est-à-dire celles qui quittent les corpuscules tendineux de Golgi. Ces afférences tendineuses étant inhibitrices des motoneurones de leur muscle, à savoir le double sourcilier, leur excitation par les impulsions électriques produit un relâchement du muscle ^{(11,12)}.

Selon l'invention, les fréquences électriques sont plus élevées (au moins 120 ou 150 Hz) et la durée d'application beaucoup plus longue (typiquement supérieure à 60 s), que dans le brevet US-A-4,957,480. Il se produit ainsi, non pas une fatigue de travail, mais une fatigue intense dite électrique (^{4,6}). L'activité électrique, caractérisée par les potentiels de repos et d'action, sur les fibres musculaires disparaît par accumulation d'ions potassium (K⁺) en extra-cellulaire. Le couplage électromécanique de l'activité musculaire est rompu par la disparition du potentiel électrique des fibres. Ceci a pour conséquence une suspension totale de l'activité mécanique du muscle et donc une relaxation complète, le muscle étant en état réversible de sidération électromécanique. L'obtention de cet état est par ailleurs évité et contre-indiqué en électro-stimulation conventionnelle. Il ne peut se produire que grâce aux paramètres électriques spécifiques de la présente invention. Il ne s'observe ni en électro-stimulation, ni lors de contractions volontaires.

### Le dispositif

Tout le système permettant l'électro-inhibition est inclus dans un dispositif unique 1.

Selon une première forme d'exécution préférée, représenté sur les figures 1 à 4, celui-ci est conçu de façon similaire à une monture de lunettes qui repose sur le nez à l'avant et sur les oreilles à l'arrière grâce à deux branches adéquates. Le circuit électronique 3 et l'alimentation 4 sous forme de batteries sont placés dans une partie frontale creuse du dispositif, au niveau de trappes 5. Les électrodes sur la face interne et médiane de la partie frontale 6 sont configurées pour se positionner correctement dans l'espace inter-sourcilier lorsque le dispositif est mis en place sur un sujet.

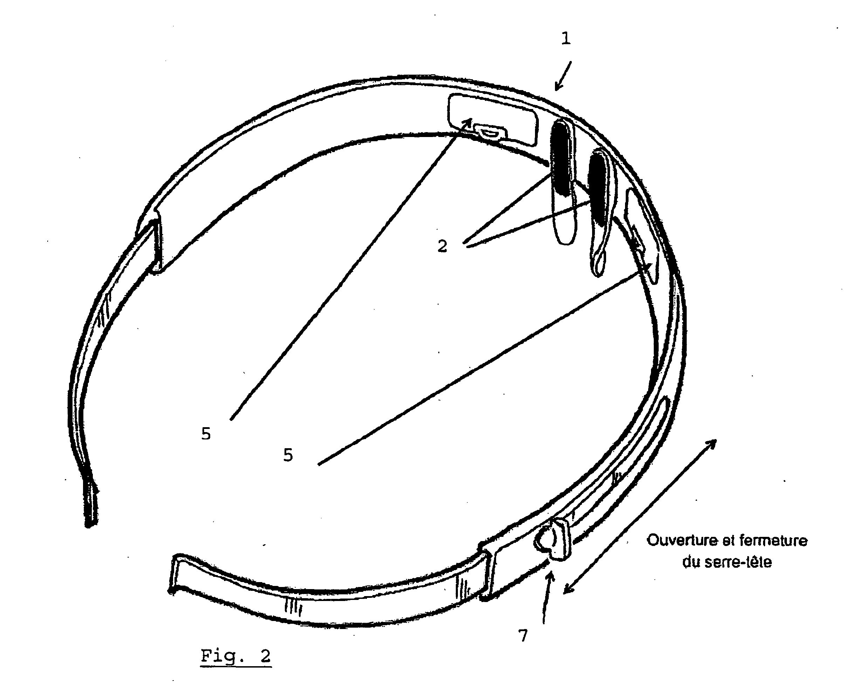

Plusieurs modalités de réalisation avantageuses de ce dispositif sont possibles, telles que :
- montage sur un serre-tête, comme représenté sur la figure 2. Dans ce cas, on peut par exemple prévoir des moyens de blocage et fixation 7, qui peuvent servir également d'interrupteur enclenchant l'alimentation électrique, une fois le serre-tête en place sur le sujet ;
- montage sur une surface autocollante venant se placer au niveau de la glabelle ;
- arcade de soutien du dispositif s'appuyant sur la ligne médiane du crâne ;
- petit casque, etc.

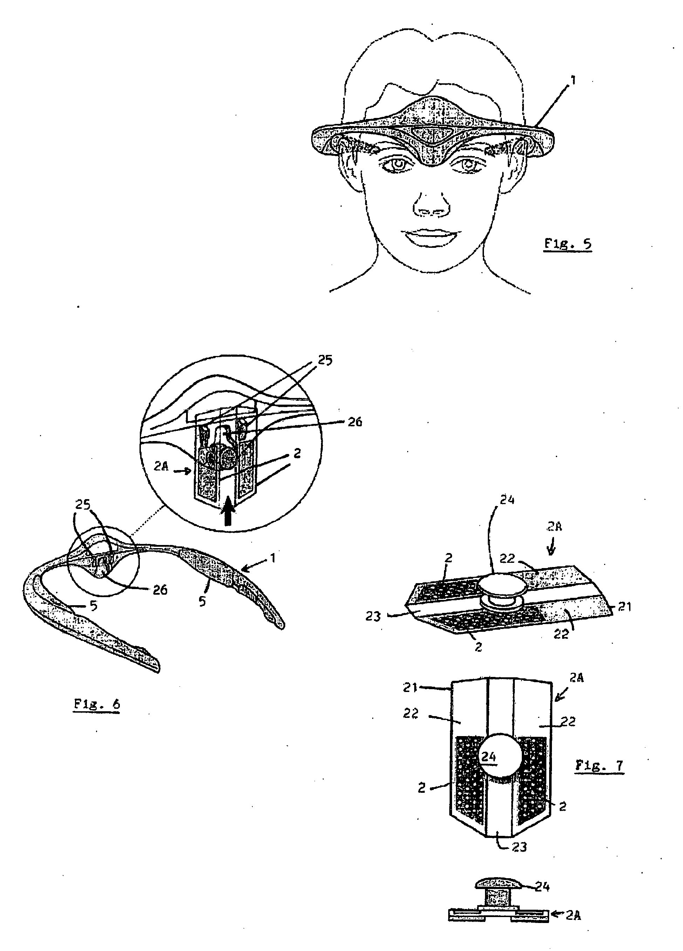

Selon une deuxième forme d'exécution préférée, représentée sur les figures 5 à 7, on a conçu une électrode "double" 2A, c'est-à-dire dans laquelle les deux pôles conducteurs 2 sont portés par une seule et même structure. Pour l'utilisateur, cette disposition présente la commodité de ne devoir placer qu'une seule électrode, plutôt que deux, comme c'est habituellement le cas dans toutes les techniques classiques d'électrothérapie.

L'électrode 2A présente une forme et une taille adaptée à la zone à traiter, c'est-à-dire qui permet de couvrir la zone de la glabelle et d'inhiber le muscle pyramidal. Selon l'invention, l'électrode 2A comprend une pièce de tissu 21 essentiellement rectangulaire, le côté le plus long devant être positionné essentiellement verticalement, et qui présente éventuellement des coins coupés au niveau de sa partie inférieure se plaçant sur le haut du nez et un côté supérieur arrondi se plaçant au niveau du front (figure 7).

La pièce de tissu 21 qui constitue le support mécanique de l'électrode est imprégné de deux zones argentées conductrices 2 qui traversent l'épaisseur du tissu. Ainsi, le tissu devient conducteur entre sa face interne, qui se place du côté de la peau et sa face externe, qui se place du côté des patins de contact au niveau de l'appareil électrique. Habituellement dans l'état de la technique, la conduction entre l'appareil électrique et les électrodes est assurée par un câble ou un bouton-pression ("snap") métallique, la surface externe de l'électrode étant par ailleurs non conductrice. Selon l'invention, les surfaces externes des pôles de l'électrode sont conductrices, ce qui permet à deux patins conducteurs 25 de l'appareil de venir en contact directement avec les pôles.

Une conduction optimale entre les zones conductices 2 et la peau de l'utilisateur est assurée par deux zones de gel conducteur autocollant 22 couvrant lesdites zones conductrices sur la face interne de l'électrode, en contact avec la peau. De préférence, ces deux zones de gel conducteur s'étendront sur toute la longueur de la pièce de tissu et, à partir du bord latéral extérieur, sur une largeur suffisante pour couvrir complètement la zone argentée correspondante 2 (figure 7).

Une alternative avantageuse à l'imprégnation à l'argent consiste à pratiquer deux ouvertures dans le tissu de l'électrode pour permettre aux patins mentionnés ci-dessus d'entrer directement en contact avec le gel conducteur ou avec une feuille de carbone, éventuellement de carbone recouvert d'une couche argentée, qui se trouve disposée entre le tissu et le gel conducteur (non représenté).

L'isolation entre les deux pôles 2 de l'électrode 2A est assurée par une zone centrale 23 dépourvue de gel conducteur auto-collant 22.

Toujours selon une modalité avantageuse de l'invention, l'électrode 2A est pourvue d'une pièce en forme de pion 24 non conductrice pour assurer l'emboîtement mécanique du dispositif de support et de l'électrode 2A et permettre aux deux patins de contact 25 de venir s'appliquer exactement sur les deux zones externes conductrices de l'électrode.

Comme décrit ci-dessus, le circuit électronique se situe dans la zone centrale du dispositif de support 1. De ce circuit sortent les deux lames ou patins de contact 25 destinés à venir s'appliquer sur les deux surfaces externes conductrices de l'électrode précitée 2A, c'est-à-dire les deux zones de l'électrode imprégnées d'argent conducteur.

Entre les deux patins de contact 25, la partie centrale du boîtier est évidée et pourvue d'une logette 26 qui permet l'emboîtement du "pion" 24 de l'électrode. Cette logette 26 est large dans sa partie inférieure et étroite dans sa partie supérieure, ceci permettant à l'utilisateur d'effectuer très facilement l'emboîtement entre l'appareil-support et l'électrode. Le pion 24 comprend en effet avantageusement une courte tige terminée par une tête ronde renflée qui lui permet de venir se bloquer dans la partie étroite de la logette 26 du dispositif de support 1 (figure 6).

Selon un exemple concret, le support en tissu 21 a comme dimensions 37 mm x 22 mm, les électrodes ont comme dimensions 21 mm x 7 mm, la zone centrale 23 entre les électrodes dépourvue de gel autocollant est large de 5 mm et le pion 24 est ancré à une distance de 16,5 mm du bord inférieur de la pièce 2A.

La stabilité du dispositif de support 1 en forme de monture de lunette est assurée par trois points, d'une part les deux branches qui reposent sur les oreilles et d'autre part l'électrode collée sur la glabelle. Ceci est différent d'une monture de lunette classique dont le troisième point de stabilité est assuré par la selle de nez ou les plaquettes qui viennent s'appuyer sur le nez. Ce dernier système ne peut convenir pour le dispositif de l'invention car les variations, d'un individu à l'autre, de la forme et de la taille du nez ne permettent pas toujours aux patins de contact de venir se placer exactement au niveau des surfaces conductrices de l'électrode.

### Avantages

Outre les avantages de simplicité et de confort déjà mentionnés ci-dessus, le dispositif de l'invention présente encore l'avantage d'être de réalisation peu coûteuse, d'utilisation très facile et brève (typiquement moins de 5 minutes), notamment au domicile de l'utilisateur et de ne pas induire les effets secondaires que l'on retrouve dans les techniques à injection (allergie, rougeurs, oedèmes, présence d'un implant artificiel, etc.).

### Références bibliographique

⁽¹⁾ J. P. REILLY, Impedance and current distribution, Applied Bioelectricity, 2nd Ed., Hardback, Springer-Verlag, New York, 1998, pp. 12-45.
⁽²⁾ L.A. GEDDES and L.E. BAKER, Electrodes, Principles of Applied Biomedical Instrumentation, 3rd Ed., John Wiley & Sons, 1989, pp. 315-387.
⁽³⁾ L.A. GEDDES and L.E. BAKER, Stimulators and Stimulation, Principles of Applied Biomedical Instrumentation, 3rd Ed., John Wiley & Sons, 1989, pp. 453-469.
⁽⁴⁾ D.A. JONES and B. BIGLAND-RITCHIE, Excitation Frequency and Muscle Fatigue : Mechanical Responses during Voluntary and stimulated Contractions, Experimental Neurology 64, pp. 401-413 (1979).
⁽⁵⁾ D.A. JONES, Muscle Fatigue due to Changes beyond the Neuromuscular Junction, Human Muscle Fatigue : Physiological Mechanisms, Pitman Medical London, 1981 (Ciba Foundation Symposium 82), pp. 178-196.
⁽⁶⁾ B. BIGLAND-RITCHIE, EMG and Fatigue of Human Voluntary and Stimulated Contractions, Human Muscle Fatigue : Physiological Mechanisms, Pitman Medical London 1981 (Ciba Foundation symposium 82), pp. 130-156.
⁽⁷⁾ D.A. JONES, High and Low-Frequency Fatigue Revisited, Acta Physiol. Scand. 156, 265-270 (1996).
⁽⁸⁾ A.C. GUYTON, Reciprocal Innervation, Textbook of Medical Physiology, 5th Edition, Sanders Company, 1976, pp. 626-27.
⁽⁹⁾ J.B. WAL, Modulation of Spasticity : Prolonged Suppression of a Spinal Reflex by Electrical Stimulation, Science 216 : 203-204, 1982.
⁽¹⁰⁾ M.G. LEVIN, M. KNOTT and H. KABAT, Relaxation of Spasticity by Electrical Stimulation of Antagonist Muscles, Arch. Phys. Med. 33 : 668-673, 1952.
⁽¹¹⁾ A.C. GUYTON, Tendon Reflex, Textbook of Medical Physiology, 5th edition, Sanders Company, 1976, pp. 623-24.
⁽¹²⁾ J. HOUK and E. HENNEMAN, Responses of Golgi tendon organs to active contractions of the soleus muscle of the cat, J. Neurophysiol. 30 : 466, 1967.

## Revendications

1. Dispositif d'électro-inhibition de muscles de la face comprenant :
- un support au niveau de la tête d'un utilisateur (1), deux électrodes de contact (2) aptes à être disposées de chaque côté de la partie supérieure du nez, au niveau de la glabelle, lesdites électrodes (2) appartenant à une structure unique d'électrode (2A),
- un circuit électronique (3) pour la création d'impulsions électriques basse tension au niveau desdites électrodes (2) et une alimentation électrique à courant continu (4),
**caractérisé en ce que** :
- la structure unique d'électrode (2A) comprend une pièce de tissu (21) servant de support auxdites électrodes (2), ces dernières étant couvertes sur leur face interne, c'est-à-dire la face devant être en contact avec la peau de l'utilisateur, par un gel conducteur autocollant (22) et
- les deux électrodes (2) se présentent sous la forme de deux zones argentées conductrices qui imprègnent ledit tissu (21), celui-ci étant conducteur aussi bien sur sa face interne que sur sa face externe, c'est-à-dire la face en contact avec des patins (25) de l'appareil électrique.

2. Dispositif selon la revendication 1, **caractérisé en ce que** deux ouvertures sont pratiquées dans la pièce de tissu (21) pour permettre auxdits patins (25) d'entrer en contact directement avec ledit gel conducteur autocollant (22) ou avec une feuille de carbone éventuellement recouverte d'une couche d'argent insérée entre le tissu et le gel conducteur.

3. Dispositif selon la revendication 1, **caractérisé en ce que** le gel conducteur (22) est déposé sous forme de zones s'étendant sur toute la longueur de la pièce en tissu (21) et s'étendant, à partir du bord latéral extérieur sur une largeur suffisante pour couvrir complètement les deux électrodes (2).

4. Dispositif selon la revendication 3, **caractérisé en ce que** la pièce de tissu (21) présente une zone centrale dépourvue de gel conducteur pour assurer l'isolation électrique entre les électrodes (2).

5. Dispositif selon la revendication 1, **caractérisé en ce que** la structure unique en tissu présente une forme essentiellement rectangulaire avec le grand côté ou longueur du rectangle disposable essentiellement verticalement et sur laquelle les électrodes (2) sont séparées l'une de l'autre par une distance comprise entre 3 et 15 mm, ladite structure présentant éventuellement des coins coupés au niveau de sa partie inférieure se plaçant sur le haut du nez et un côté supérieur arrondi se plaçant au niveau du front.

6. Dispositif selon l'une quelconque des revendications 1 à 5, **caractérisée en ce que** la structure unique d'électrode (2A) est pourvue d'une pièce en forme de pion (24) non conductrice pour assurer l'emboîtement mécanique de ladite structure (2A) dans ledit support (1) avec mise en contact desdits patins (25) avec les électrodes (2) respectives.

7. Dispositif selon la revendication 6, **caractérisé en ce que** le pion (24) comprend une courte tige surmontée d'une tête ronde renflée, apte à venir se bloquer dans la partie étroite d'une logette (26) du support (1), plus large dans sa partie inférieure que dans sa partie supérieure.

8. Dispositif selon l'une quelconque des revendications 1 à 7, **caractérisé en ce qu'**il comprend des moyens de fixation et blocage (7) du type monture de lunettes avec un appui à l'avant sur le nez au niveau de la structure unique d'électrode (2A) et deux branches qui se positionnent derrière les oreilles.

9. Dispositif selon l'une quelconque des revendications 1 à 7, **caractérisé en ce qu'**il comprend des moyens de fixation et blocage (7) sous forme d'un serre-tête destiné à se positionner au niveau du front.

10. Dispositif selon l'une quelconque des revendications 1 à 7, **caractérisé en ce qu'**il comprend des moyens de fixation et blocage (7) sous forme d'une arcade de soutien s'appuyant sur la ligne médiane du crâne.

11. Dispositif selon l'une quelconque des revendications 1 à 7, **caractérisé en ce qu'**il comprend des moyens de fixation et blocage (7) sous forme d'un casque en matière légère.

12. Dispositif selon l'une quelconque des revendications 8 à 11, **caractérisé en ce que** les moyens de blocage sont configurés pour que, en position verrouillée, respectivement en position déverrouillée, le système fait également office d'interrupteur fermé, respectivement ouvert, dudit circuit électronique (3).

## Patentansprüche

1. Vorrichtung für die Elektro-Inhibition der Gesichtsmuskeln, umfassend:
- eine Armatur für den Kopf eines Benutzers (1) mit zwei zur Anordnung auf jeder Seite des oberen Nasenbereichs im Bereich der Glabella geeignete Kontaktelektroden (2), welche zu einer einzigen Elektrodenstruktur gehören (2A),
- eine elektronische Schaltung (3) zur Erzeugung elektrischer Impulse mit niedriger Spannung an den Elektroden (2) sowie eine Gleichstrom-Spannungsversorgung (4),
**dadurch gekennzeichnet, dass**:
- die Elektrodenstruktur (2A) ein Stoffstück (21) als Träger der Elektroden (2) umfasst, wobei letztere auf der Innenseite, d.h. die Seite, die mit der Haut des Benutzers in Berührung kommen soll, durch ein selbstklebendes leitfähiges Gel (22) beschichtet sind, und
- die beiden Elektroden (2) sich als zwei leitfähige versilberte Bereiche darstellen, die das Stoffstück (21) imprägnieren, wobei dieses sowohl auf der Innen- als auch auf der Außenseite, d.h. auf der die Kontaktlinsen (25) der Vorrichtung berührenden Seite, leitfähig ist.

2. Vorrichtung nach Anspruch 1, **dadurch gekennzeichnet, dass** im Stoffstück (21) zwei Öffnungen vorgesehen sind, die es den Kontaktlinsen (25) ermöglichen, unmittelbar mit dem leitfähigen Gel (22) oder einer zwischen dem Stoff und dem Gel angeordneten, evtl. mit einer Silberschicht beschichteten Kohlenstoffschicht in Berührung zu treten.

3. Vorrichtung nach Anspruch 1, **dadurch gekennzeichnet, dass** das leitfähige Gel (22) in Gestalt von Bereichen, die sich über die ganze Länge des Stoffstückes (21) sowie vom lateralen Außenrand über eine zur vollständigen Beschichtung der beiden Elektroden (2) ausreichende Länge erstrecken, aufgetragen wird.

4. Vorrichtung nach Anspruch 3, **dadurch gekennzeichnet, dass** das Stoffstück (21) einen nicht mit leitfähigem Gel beschichteten Mittelbereich aufweist, um die Elektroisolierung zwischen den Elektroden (2) zu gewährleisten.

5. Vorrichtung nach Anspruch 1, **dadurch gekennzeichnet, dass** die einzige Stoffstruktur eine im Wesentlichen rechteckige Form aufweist, wobei die Längsseite bzw. die Länge des Rechtecks im Wesentlichen senkrecht angeordnet werden und auf der die Elektroden (2) durch einen Abstand von 3 - 15 mm voneinander getrennt sind, wobei die Struktur eventuell abgeschräge Ecken im unteren Bereich, der auf der Oberseite der Nase angebracht wird und eine abgerundete Oberseite, die auf der Stirn angebracht wird, aufweist.

6. Vorrichtung nach einem der Ansprüche 1 - 5, **dadurch gekennzeichnet, dass** die einzige Elektrodenstruktur (2A) mit einem Zylinderförmigen, nichtleitfähigen Stück (24) versehen ist, um das mechanische Ineinandergreifen der Struktur (2A) in die Armatur (1) unter Inberührungbringen der Kontaktlinsen (25) mit den jeweiligen Elektroden (2) zu gewährleisten.

7. Vorrichtung nach Anspruch 6, **dadurch gekennzeichnet, dass** das Zylinderförmige Stück (24) eine kurze Stange, auf der ein rundes, konvexes Kopfstück montiert ist, aufweist, die geeignet ist, im engen Teil eines Gehäuses (26) der Armatur (1) fixiert zu werden, dessen Unterteil breiter als sein Oberteil ist.

8. Vorrichtung nach einem der Ansprüche 1 - 7, **dadurch gekennzeichnet, dass** sie Fixier- und Blockiermittel (7) in Gestalt einer Brillenfassung mit einer VorderArmatur auf der Nase im Bereich der einzigen Elektrodenstruktur (2A) und zwei hinter den Ohren angeordnete Bügel umfasst.

9. Vorrichtung nach einem der Ansprüche 1 - 7, **dadurch gekennzeichnet, dass** sie Fixier- und Blockiermittel (7) in Gestalt eines Haarreifes zur Positionierung auf der Stirn umfasst.

10. Vorrichtung nach einem der Ansprüche 1 - 7, **dadurch gekennzeichnet, dass** sie Fixier- und Blockiermittel (7) in Gestalt einer bogenförmigen Armatur, die sich auf der Mittellinie des Schädels abstützt, umfasst.

11. Vorrichtung nach einem der Ansprüche 1 - 7, **dadurch gekennzeichnet, dass** sie Fixier- und Blockiermittel (7) in Gestalt eines Helms aus leichtem Stoff umfasst.

12. Vorrichtung nach einem der Ansprüche 8 - 11, **dadurch gekennzeichnet, dass** die Blockiermittel derart angeordnet sind, dass, wenn sie sich in der geschlossenen bzw. offenen Position befinden, das System auch als geschlossener bzw. offener Schalter der elektronischen Schaltung (3) fungiert.

## Claims

1. Apparatus for the electro-inhibition of the face muscles comprising:
- a support at the level of a user's head (1), two contact electrodes (2) to be positioned on either side of the upper part of the nose at the level of the glabella, said electrodes (2) belonging to a single electrode structure (2A),
- an electronic circuit (3) for generating low-voltage electric pulses at the level of said electrodes (2) and a direct-current power supply (4),
**characterized in that**:
- the single electrode structure (2A) comprises a piece of cloth (21) acting as a support for both electrodes (2), the latter being covered on their inner face, i.e. the face that must be in contact with the user's skin, by a self-adhesive conductive gel (22) and
- both electrodes (2) are in the shape of two conductive silvered areas that impregnate said cloth (21), the latter being conductive on both its inner and outer faces, i.e. the faces in contact with the pads (25) of the electric device.

2. Apparatus according to Claim 1, **characterized in that** two holes are made in the piece of cloth (21) to allow said pads (25) to come into direct contact with said self-adhesive conductive gel (22) or with a carbon film, possibly covered with a layer of silver inserted between the cloth and the conductive gel.

3. Apparatus according to Claim 1, **characterized in that** the conductive gel (22) is applied in the form of areas extending over the whole length of the piece of cloth (21) and extending, from the outer lateral edge over a width that is sufficient to completely cover both electrodes (2).

4. Apparatus according to Claim 3, **characterized in that** the piece of cloth (21) has a central area devoid of any conductive gel in order to ensure the electric insulation between the electrodes (2).

5. Apparatus according to Claim 1, **characterized in that** the single structure made of cloth has an essentially rectangular shape with the long side or length of the rectangle arranged essentially vertically and on which the electrodes (2) are separated from each other by a distance between 3 and 15mm, said structure possibly having its corners cut at the level of its lower part being placed on the top of the nose and a rounded upper side being placed at the level of the forehead.

6. Apparatus according to any one of Claims 1 to 5, **characterized in that** the single electrode structure (2A) is provided with a non-conductive part in the shape of a pin (24) to ensure the mechanical fitting of said structure (2A) into said support (1) with said pads (25) being brought into contact with the respective electrodes (2).

7. Apparatus according to Claim 6, **characterized in that** the pin (24) comprises a short stem topped by a bulging round head which can block into the narrow part of a recess (26) of the support (1) which is wider in its bottom part than in its upper part.

8. Apparatus according to any one of Claims 1 to 7, **characterized in that** it comprises attaching and securing (7) means of a spectacle frame type resting at the front on the nose at the level of the single electrode structure (2A) and two arms that are positioned over the ears.

9. Apparatus according to any one of Claims 1 to 7, **characterized in that** it comprises attaching and securing (7) means in the shape of a headband intended to be positioned at the level of the forehead.

10. Apparatus according to any one of Claims 1 to 7, **characterized in that** it comprises attaching and securing (7) means in the shape of a supporting hoop resting on the medial line of the skull.

11. Apparatus according to any one of Claims 1 to 7, **characterized in that** it comprises attaching and securing (7) means in the shape of a helmet made of light material.

12. Apparatus according to any of Claims 8 to 11, **characterized in that** the securing means is configured so that, in locked and unlocked position respectively, the system acts both as a closed and open switch respectively of said electronic circuit (3).
